# EUROPÄISCHE PATENTSCHRIFT

(11) **EP 2 632 392 B1**
(45) Veröffentlichungstag und Bekanntmachung des Hinweises auf die Patenterteilung: **03.06.2015**
(21) Anmeldenummer: 11818985.1
(22) Anmeldetag: 25.10.2011
(51) Int. Cl.: A61F 2/66, A61F 2/50

(54) **PROTHESENFUSS**
PROSTHETIC FOOT
PIED PROTHÉTIQUE

(30) Priorität: 08.11.2010 DE 102010050493; 25.10.2010 DE 102010049257
(43) Veröffentlichungstag der Anmeldung: 04.09.2013
(73) Patentinhaber: Otto Bock HealthCare GmbH, 37115 Duderstadt (DE)
(72) Erfinder: MOSLER, Lüder, 37115 Duderstadt (DE)
(74) Vertreter: Stornebel, Kai
(86) Internationale Anmeldenummer: PCT/DE2011/001941
(87) Internationale Veröffentlichungsnummer: WO 2012/062279

(56) Entgegenhaltungen:
- WO-A1-2004/032809
- US-A1- 2002 087 216
- US-A1- 2010 023 135

## Beschreibung

Die Erfindung betrifft einen Prothesenfuß mit einer Basisfeder mit einem Vorderfußbereich und einem Fersenbereich, einer oberhalb der Basisfeder angeordneten Abschlusseinrichtung zur Befestigung des Prothesenfußes an einer Prothese und einer Frontalabstützung, an deren oberen Ende die Anschlusseinrichtung angeordnet und an ihrem unteren Ende an einem um die Prothesenlängsachse tordierbaren Torsionselement festgelegt ist. Die Erfindung betrifft ebenfalls einen Prothesenfuß mit einer Basisfeder mit einem Vorderfußbereich und einem Fersenbereich sowie einer oberhalb der Basisfeder angeordneten Anschlusseinrichtung zur Befestigung des Prothesenfußes an einer Prothese.

Die DE 10 2006 004 132 A1 beschreibt einen künstlichen Fuß mit einer sich von einem Fersenbereich zu einem Zehenbereich erstreckenden elastischen Sohlenstruktur mit einer Längsachse, einem Anschluss zu einem Unterschenkelteil, einer sich in die Höhe erstreckenden oberen Stützstruktur und einem zwischen der oberen Stützstruktur und der Sohlenstruktur angeordneten elastischen Verbindungselement. Die obere Stützstruktur ist etwa in der in Längsrichtung etwa in der Mitte des Fußes mit der Sohlenstruktur über eine Kupplungsanordnung verbunden, die eine relative Kippbewegung zwischen der Sohlenstruktur und der Stützstruktur erlaubt. Dabei bleibt der Abstand zwischen der Stützstruktur und der Sohlenstruktur in der Mitte der Kupplungsanordnung zumindest bei einer Gewichtsbelastung im Stand der Patienten konstant. Die Gelenkanordnung besteht aus einem elastischen Kunststoffzylinder, der sich mit einer unteren Stirnfläche an der Sohlenstruktur und einer oberen Stirnfläche an der Unterseite der Stützstruktur abstützt. Eine Stiftanordnung ergänzt den Kunststoffzylinder und weist an ihren beiden Enden Kugelköpfe auf, die sich an der Sohlenstruktur einerseits und an der Stützstruktur andererseits abstützen und die Stabilität der Verbindung erhöhen. Es ist ebenfalls möglich, dass die Gelenkanordnung in Form einer Kugelkalotte mit einer entsprechend zusammenwirkenden Kugelpfanne ausgebildet ist, um so die kippbare Verbindung zwischen der Stützstruktur und der Sohlenstruktur herzustellen.

Die US 2010/023135 A1 betrifft einen Prothesenfußeinsatz mit einer Basisfeder, einer kurvenförmig gebogenen Feder, an deren proximalem Höchstniveau eine Anschlusseinrichtung zur Befestigung an ein Unterschenkelrohr vorgesehen ist. Die kurvenförmige Feder erstreckt sich sowohl frontal als auch distal in Richtung der Basisfeder und des Vorderfußes. Ein Blattfederelement erstreckt sich von dem Vorderfußbereich in Richtung des Endes der kurvenförmigen Feder und liegt dort an.

Die WO 2004/032809 A1 betrifft einen Prothesenfuß mit einer Basisfeder mit einem Vorderfußbereich und einem Fersenbereich. Von dem Fersenbereich erstreckt sich eine S-förmig gebogene Fersenfeder bis zu einer proximal angeordneten Anschlusseinrichtung an einer proximalen Protheseneinrichtung. Von der Anschlusseinrichtung erstreckt sich eine C-förmige Feder bis zum Vorderfußbereich der Basisfeder. Zwischen der Basisfeder und der C-förmigen Feder ist ein Polsterelement angeordnet, im Fersenbereich der S-förmigen Fersenfeder sind ebenfalls Polsterelemente angeordnet

Die US 2002/087216 A1 betrifft ein prothetisches Fußsystem mit einer Basisfeder, einer U-förmig nach hinten offenen Feder, deren untere Schenkel auf der Basisfeder aufliegt und an deren oberen Schenkel ein Pylon befestigt ist. Zwischen den Schenkeln Ist am fersenseitigen Ende ein Polster angeordnet, um Fersenstöße abzufangen. Zur Begrenzung der Aufweitbewegung ist ein Band zwischen den freien Schenkeln angeordnet.

Mit dem künstlichen Fuß gemäß dem Stand der Technik ist eine verbesserte Abrollbarkeit während des Gehens möglich, darüber hinaus ist eine gut definierte Statik des Stehens durch eine nach vorn verlagerten Krafteinleitungspunkt vorhanden. Durch den elastischen Kunststoffzylinder sind jedoch nur geringe Rückstellkräfte realisierbar, so dass die Federeigenschaften des künstlichen Fußes beim Gehen durch die Sohlenstruktur allein realisiert
werden muss. Darüber hinaus werden mit dem Kunststoffzylinder verschleißanfällige Komponenten eingesetzt, was der Präzision der Krafteinleitung abträglich ist.

Aufgabe der vorliegenden Erfindung ist es, einen Prothesenfuß bereitzustellen, der verbesserte Möglichkeiten zur Abstimmung des Abrollverhaltens des Prothesenfußes und eine präzise Statik während des Stehens auch bei variablen Untergründen bietet.

Erfindungsgemäß wird diese Aufgabe durch einen Prothesenfuß mit den Merkmalen des Hauptanspruches oder des Nebenanspruches gelöst. Vorteilhafte Ausgestaltungen und Weiterbildungen sind in den Unteransprüchen der Beschreibung und den Figuren aufgeführt.

Der erfindungsgemäße Prothesenfuß mit einer Basisfeder mit einem Vorderfußbereich und einem Fersenbereich, einer oberhalb der Basisfeder angeordneten Anschlusseinrichtung zur Befestigung des Prothesenfußes an einer Prothese und einer Frontalabstützung, an deren oberen Ende die Anschlusseinrichtung angeordnet und an ihrem unteren Ende an einem um die Prothesenfußlängsachse tordierbaren Torsionselement festgelegt ist, sieht vor, dass das Torsionselement als eine einseitig im Fersenbereich oder im Vorfußbereich festgelegte Blattfedereinrichtung ausgebildet ist. Durch das einseitige Einspannen des Torsionselementes im Fersenbereich ist es möglich, dass beim Aufsetzen des Fersenbereiches neben einer Torsion um die Fußlängsachse auch eine Biegung um eine quer zur Fußlängsachse verlaufende Achse erfolgen kann, so dass das Torsionselement neben der Möglichkeit eines Winkelausgleiches in Gehrichtung auch ein zusätzliches Federelement bereitstellt, um einen relativ sanften Fersenauftritt zu ermöglichen. Nach dem Aufsetzen des Vorderfußbereiches und während des Abrollens des Prothesenfußes liegt dann das freie, vordere Ende des Torsionselementes auf der Basisfeder auf, so dass sich eine Versteifung durch eine Parallelschaltung der Basisfeder und des Torsionselementes ergibt. Mit dem erfindungsgemäßen Prothesenfuß ist es möglich, die gut definierte Statik über den vorverlagerten Krafteinleitungspunkt in eine dynamische Konstruktion zu überführen und dabei gleichzeitig die Standstabilität auch bei wechselnden Untergründen oder verschiedenen Schuhen zu gewährleisten.

Über das Torsionselement, das in der Regel eine Verlagerbarkeit oder Verformbarkeit in Längserstreckung des Prothesenfußes verhindert, wird der Auflagepunkt der Stützstruktur auf der Sohlenstruktur präzise ortsständig gehalten, ohne das ein verschleißbedingte Veränderung der Krafteinleitungsposition erfolgen kann. An der Anschlusseinrichtung oder an dem oberen Ende der Frontalabstützung ist ein Dämpferelement oder ein nachgiebiges Element angeordnet, das sich im Fersenbereich der Basisfeder an der Basisfeder oder dem Torsionselement abstützt. Eine solche Ausgestaltung ist insbesondere dann vorgesehen, wenn das Torsionselement einseitig im Fersenbereich festgelegt ist. Dadurch kann eine zusätzliche Dämpfung des Fersenstoßes erfolgen. Das Dämpferelement kann als Fluiddämpfer, insbesondere als Hydraulikdämpfer oder Pneumatikdämpfer ausgebildet sein, um eine leichte Einstellbarkeit des Dämpfungsgrades erreichen zu können. Fluiddämpfer können sowohl in der Zugstufe als auch in der Druckstufe separat eingestellt werden, so dass ein variables Einfederverhalten und Ausfederverhalten der Anschlusseinrichtung bzw. des Dämpferkolbens gewährleistet werden kann. Zusätzlich zur besseren Einstellbarkeit verfügt der Dämpfer über die Eigenschaft, statisch keine Kräfte aufzubauen. Der Fuß kann sich somit im Stehen immer wieder an den Untergrund oder das Schuhwerk anpassen, es wirken lediglich die Rückstellkräfte der relativ biegeweichen Torsionsfeder.

In einer alternativen Ausgestaltung ist vorgesehen, dass der Prothesenfuß mit einer Basisfeder mit einem Vorderfußbereich und einem Fersenbereich sowie einer oberhalb der Basisfeder angeordneten Anschlusseinrichtung zur Befestigung des Prothesenfußes an einer Prothese, ausgestattet ist, wobei die Anschlusseinrichtung an einem um die Prothesenfußlängsachse tordierbaren Torsionselement festgelegt ist, das als eine einseitig im Fersenbereich an der Basisfeder festgelegte Blattfedereinrichtung ausgebildet ist, wobei das Torsionselement parallel zur Basisfeder geschaltet ist und bei einer Vorfußbelastung auf der Oberseite der Basisfeder aufliegt. Das Torsionselement als Blattfeder ist ausschließlich im Fersenbereich der Basisfeder befestigt, so dass bei einem Fersenauftritt das vordere Ende des Torsionselementes sich von der Basisfeder entfernt und den Abstand im vorderen Bereich des Torsionselementes zu der Basisfeder vergrößert Die Anschlusseinrichtung ist dabei vor der Befestigungsstelle des Torsionselementes an der Basisfeder angeordnet, so dass das Torsionselement bei einem Fersenauftritt ebenfalls federnd mitwirkt. Im Verlauf der weiteren Standphase senkt sich die Basisfeder zu einem vollständigen Kontakt auf dem Boden ab. Im Anschluss daran wird das Torsionselement weiter in Richtung auf die Basisfeder abgesenkt, bis das vordere Ende des Torsionselementes auf der Oberseite der Basisfeder aufliegt. Bei zunehmender Vorfußbelastung biegen sich sowohl die Basisfeder als auch das Torsionselement gemeinsam in Richtung auf den Boden durch, so dass die Basisfeder und das Torsionselement gemeinsam wirken und parallel geschaltet sind. Das Torsionselement liegt dabei auf der Oberseite der Basisfeder auf und die am hinteren Ende des Torsionselementes wirksamen Kräfte werden in den Befestigungsmitteln, z.B. Schrauben, mit denen das Torsionselement an der Basisfeder befestigt ist, an die Basisfeder weitergeleitet. Um ein hartes Auftreffen des vorderen Teils des Torsionselementes auf der Basisfeder zu vermeiden, ist ein Polsterelement vorgesehen, beispielsweise ein Schaumstoff oder dergleichen. Durch die einseitige Festlegung des Torsionselementes im Fersenbereich ist sowohl eine Tordierbarkeit um die Fußlängsachse als auch eine Federung um eine medial-lateral verlaufende Achse möglich, so dass eine einfache Anpassung der Basisfeder auch an unebene Untergründe während des Stehens, also während der Standphase, erreicht werden kann.

Bei einer einseitigen Anlenkung des Torsionselementes im Vorderfußbereich kommt es zu einer Parallelschaltung der Basisfeder und des Torsionselementes bei einem Fersenauftritt, so dass ein stabiler Auftritt gewährleistet ist. Im Verlauf der Abrollbewegung kann bei zunehmender Vorfußbelastung das Torsionselement um eine Achse quer zur Längsrichtung aufgebogen werden, so dass eine zusätzliche Feder zur Abstimmung der Elastizität des Prothesenfußes vorhanden ist. Die Tordierbarkeit des Torsionselementes ändert sich während der Standphase, je nach Ort der Anlenkung unterschiedlich.

Durch die einseitige Anlenkung des Torsionselementes kann sich das andere, nicht an der Basisfeder festgelegte Ende bei einer Biegebelastung von der Basisfeder entfernen, also den Abstand zwischen der Basisfeder und dem Torsionselement vergrößern, oder auch verringern, wenn in der Ausgangsstellung ein Verlagerungsweg in Richtung auf die Basisfeder vorhanden ist.

Zwischen der Basisfeder und dem Torsionselement kann ein Freiraum ausgebildet sein, um einerseits eine Verlagerung in Richtung auf die Basisfeder zu ermöglichen und andererseits bei einem Absatz oder Auflager zwischen der Basisfeder und dem Torsionselement auch im angelegten Zustand des Torsionselementes auf dem Absatz oder Auflager eine zusätzliche Biegung des Torsionselementes bereitzustellen.

Zwischen dem Torsionselement und der Basisfeder kann ein Pufferelement angeordnet sein, um das Auftreffen des Torsionselementes auf der Basisfeder während des Abrollens oder Auftretens zu dämpfen. Das Pufferelement kann eine Form aufweisen, die eine Torsion des Torsionselementes auch im angelegten Zustand an die Basisfeder erlaubt, beispielsweise gerundet oder als ein Steg, so dass eine Verkippung des Torsionselementes relativ zu der Basisfeder möglich ist. Alternativ dazu kann die Kontaktfläche zwischen dem Torsionselement und der Basisfeder eben ausgebildet sein, so dass bei der Anlage des Torsionselementes an der Basisfeder während der Standphase keine Torsion möglich ist. Dadurch wird eine größere Stabilität des Prothesenfußes bereitgestellt.

Aufgrund der Ausgestaltung des Torsionselementes als eine Blattfedereinrichtung, also als eine einteilige oder mehrteilige Blattfeder, ist es vorgesehen, dass das Torsionselement um eine medial-lateral verlaufende Achse tordierbar ist, so dass eine gestufte Wirksamwerdung der einzelnen Federelemente, nämlich des Torsionselementes und der Basisfeder eintritt.

Die Frontalabstützung, die von der Anschlusseinrichtung, beispielsweise einem Anschlussadapter, zu dem Torsionselement führt, kann ebenfalls als eine Feder oder als ein Federpaket ausgebildet sein, um eine zusätzliche Federkomponente bereitzustellen, über die eine Anpassung an den jeweiligen Prothesenfußnutzer und an das jeweilige Einsatzgebiet oder die Vorlieben des Prothesenfußnutzers eingestellt werden kann. Vorzugsweise ist die Frontalabstützung als eine Blattfeder oder als Blattfederpaket ausgebildet.

Die Frontalabstützung kann um eine Vertikalachse tordierbar ausgebildet sein, so dass in der Standphase auch bei einer Drehbelastung um eine Vertikalachse eine gewisse Nachgiebigkeit vorhanden ist, so dass auch bei einem vollständigen Kontakt des Prothesenfußes mit dem Untergrund eine Verdrehbarkeit und Nachgiebigkeit um eine Vertikalachse möglich ist. Dadurch kann das Verhalten des Prothesenfußes während des Gehens oder Stehens für einen Prothesenfußnutzer angenehmer gestaltet werden.

Die Frontalabstützung endet in Gehrichtung bevorzugt vor der Anschlusseinrichtung, also ungefähr in dem Bereich, durch den der Kraftvektor der Bodenreaktionskraft während des Stehens durch den Prothesenfuß hindurch läuft. Die Frontalabstützung endet daher ungefähr drei bis fünf Zentimeter anterior der Anschlusseinrichtung.

Die Frontalabstützung kann sich auf der Basisfeder in einem Abstützbereich abstützen, der in Gehrichtung vor der Anschlusseinrichtung liegt Der Abstützbereich kann durch das Torsionselement oder durch Stege, Polster, Dämpfer oder dergleichen unterhalb des Torsionselementes oder auf der Basisfeder ausgestattet sein. Die Frontalabstützung kann sich in der Mitte der Basisfeder auf der Basisfeder abstützen, wobei sowohl in Längsrichtung als auch in Querrichtung eine mittige Abstützung vorteilhaft ist.

An der Basisfeder kann eine Fersenfeder angeordnet sein, die als zusätzliches Federelement eine Kraftaufnahme während des Fersenstoßes ermöglicht. Zwischen der Basisfeder und der Fersenfeder kann ein Dämpferelement angeordnet sein, um die kinetische Energie beim Auftreten in Verformungsenergie oder Wärme umzuwandeln.

Das Dämpferelement weist zumindest ein mehrachsig bewegliches Gelenk auf, so dass neben einer Verschwenkung um eine Medial-Lateral-Achse beim Einfedern auch eine Verdrehung beispielsweise um eine Anterior-Posterior-Achse erfolgen kann, um eine Torsion des Torsionselementes und damit ein Verkippen der Anschlusseinrichtung relativ zu der Basisfeder mitmachen zu können. Das mehrachsige Gelenk ist vorzugsweise an einer Lagerungsstelle an der Anschlusseinrichtung oder der Basisfeder ausgebildet, so dass das Dämpferelement selbst eine stabile lineare Bewegung ausführen kann.

Nachfolgend wird ein Ausführungsbeispiel der Erfindung anhand der beigefügten Figuren näher erläutert. Gleiche Bauteile werden mit gleichen Bezugszeichen gekennzeichnet. Es zeigen:
- Figur 1: eine perspektivische Gesamtansicht eines Prothesenfußes;
- Figur 2: eine Seitenansicht mit einer teilgeschnittenen Ferse;
- Figur 3: eine Schnittdarstellung entlang B-B in Figur 2;
- Figur 4: eine Seitenansicht einer Variante; sowie
- Figur 5: ein nicht erfindungs̈gemäßen Variante des Prothesenfußes ohne Frontalabstützung.

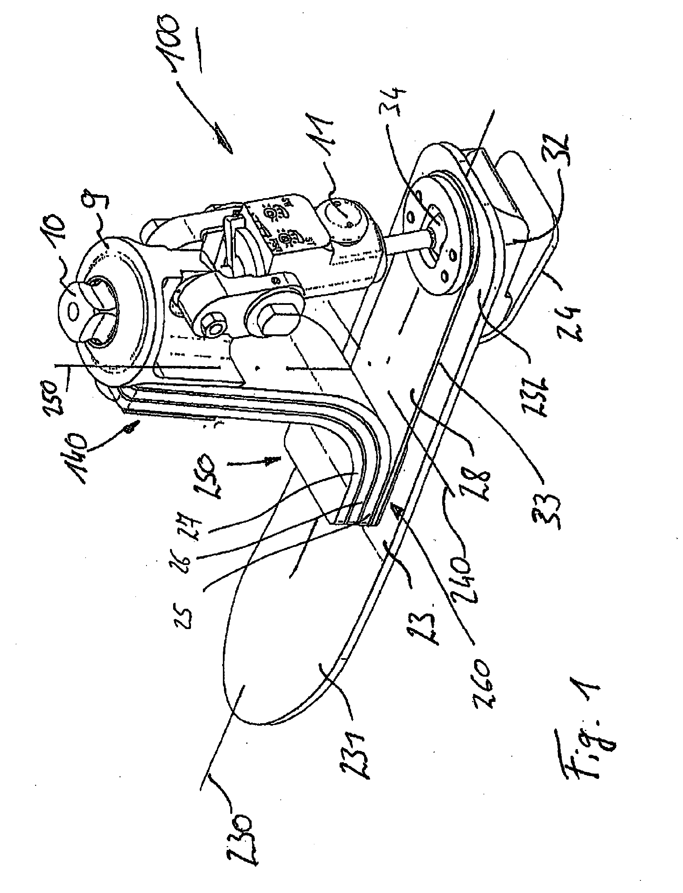

In der Figur 1 ist ein Prothesenfuß 100 in einer perspektivischen Schrägdraufsicht gezeigt. Der Prothesenfuß 100 weist eine Basisfeder 23 mit einem Vorderfußbereich 231 und einem Fersenbereich 232 auf. Die Basisfeder 23 ist bevorzugt aus einem Faserverbundwerkstoff hergestellt, insbesondere einem Carbonfaserbauteil. Die Basisfeder 23 ist langgestreckt ausgebildet, wobei in Längsrichtung die Prothesenfußlängsachse 230 verläuft. Die Basisfeder 23 ist im Wesentlichen gerade ausgebildet, im Vorderfußbereich 231 ist eine leichte Krümmung vorgesehen, um ein Abrollen des Prothesenfußes 100 zu erleichtern.

Im Fersenbereich 232 ist eine Fersenfeder 24 an der Basisfeder 23 angeordnet. Die Fersenfeder 24 ist ebenfalls aus einem Faserverbundwerkstoff ausgebildet, insbesondere aus einem Carbonfasermaterial. Die Fersenfeder 24 kann auswechselbar an der Basisfeder 23 befestigt sein. Zwischen der Fersenfeder 24 und der Basisfeder 23 ist ein sich in Gehrichtung verjüngender Freiraum vorgesehen, in dem ein Dämpferelement 32 angeordnet ist. Über dieses Dämpferelement kann auch die Fersenfeder 24 an der Basisfeder 23 festgelegt werden.

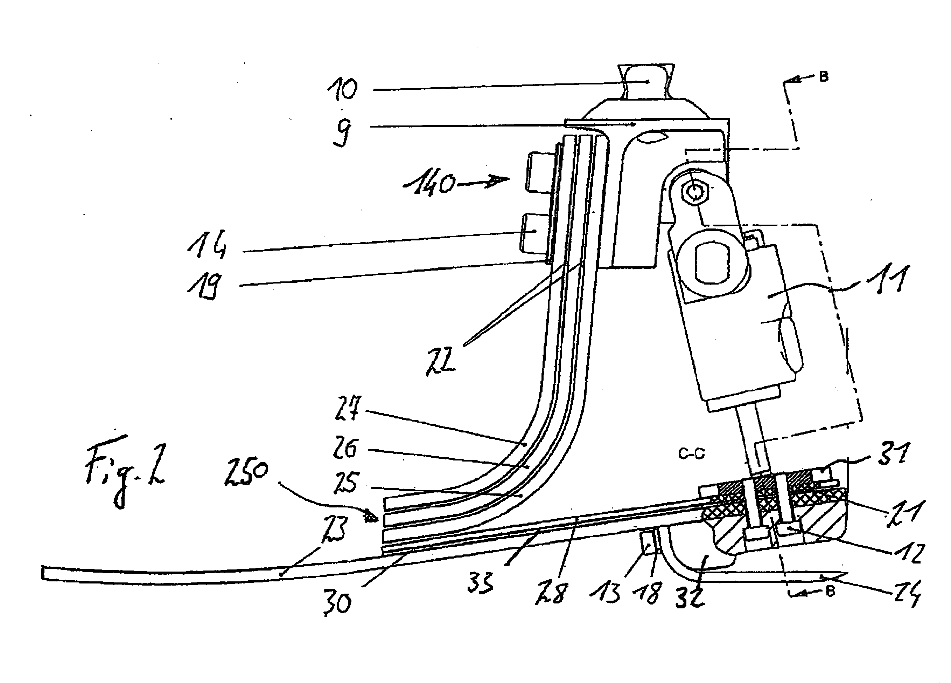

An der Oberseite der Basisfeder 23 ist ein Torsionselement 28 in Gestalt einer Blattfeder angeordnet. Das Torsionselement 28 ist im Fersenbereich 232 der Basisfeder 23 festgelegt, die Art und Weise der Festlegung wird anhand der Figur 2 erklärt werden. Das Torsionselement 28 ist lediglich einseitig an der Basisfeder 23 befestigt, das in Gehrichtung vordere Ende des Torsionselementes 28 ist nicht festgelegt und kann daher auf die Basisfeder 23 zu und von der Basisfeder 23 weg bewegt werden.

Das vordere Ende des Torsionselementes 28 endet in einem Abstützbereich 260, der ungefähr in der Mitte der Länge der Basisfeder 23 angeordnet ist. Das Torsionselement 240 ist um die Prothesenfußlängsachse 230 tordierbar, ebenfalls ist sie um eine medial-lateral verlaufende Torsionsachse 240 biegbar, so dass das vordere Ende des Torsionselementes 28 von der Oberseite der Basisfeder 23 weggebogen werden kann. Zwischen dem Torsionselement 28 und der Basisfeder 23 ist ein Freiraum 33 angeordnet, der auch im angelegten Zustand des vorderen Endes des Torsionselementes 28 auf der Basisfeder 23 eine geringfügige Durchbiegung ermöglicht.

An dem vorderen Ende des Torsionselementes 28 ist eine Frontalabstützung 25, 26, 27 in Gestalt eines Blattfederpaketes befestigt. Die Befestigung kann durch Verkleben, Verschrauben, Einlaminieren oder dergleichen erfolgen. Das untere Ende 250 der Frontalabstützung 25, 26, 27 verläuft im Wesentlichen parallel zu dem Torsionselement 28. Von der Befestigungsstelle an dem Torsionselement 28, an dem die Frontalabstützung 25, 26, 27 starr festgelegt ist, verläuft die Frontalabstützung 25, 26, 27 mit einem Bogen nach oben in Richtung auf eine Anschlusseinrichtung, die einen Proximaladapter 9 und einen Justierkern 10 aufweist. Das obere Ende 140 der Frontalabstützung 25, 26, 27 ist gerade ausgebildet und an dem Proximaladapter 9 als Teil der Anschlusseinrichtung befestigt, das distale Ende der Anschlusseinrichtung wird von einem Justierkern 10 ausgebildet und dient zur Festlegung an einer Unterschenkelprothese. Die Frontalabstützung 25, 26, 27 ist anterior an dem Proximaladapter 9 befestigt, auch hier liegt eine im Wesentlichen starre Verbindung zu der Frontalabstützung 25, 26, 27 vor.

Auf der der Frontalabstützung 25, 26, 27 gegenüberliegenden Seite des Justierkerns 10 ist ein Hydraulikdämpfer 11 angeordnet, der gelenkig an dem Proximaladapter 9 befestigt ist. Die Schwenkachse der oberen Lagerung verläuft im Wesentlichen parallel zu der Medial-Lateral-Achse 240. Am unteren Ende des Dämpfers 11 ist eine Lagerung in einem mehrachsigen, beweglichen Gelenk 34 vorgesehen, im Ausführungsbeispiel ein Teilkugelgelenk, mit dem es möglich ist, sowohl eine Verschwenkung um eine Medial-Lateral-Achse als auch eine Verdrehung um die Prothesenfußlängsachse 230 auszuführen. Aufgrund der mehrachsigen Lagerung in dem unteren Gelenk 34 und der rotationssymmetrischen Ausgestaltung des beweglichen Kolbens oder der Kolbenstange ist es auch möglich, dass eine Torsion um eine Vertikalachse 250 durchgeführt wird.

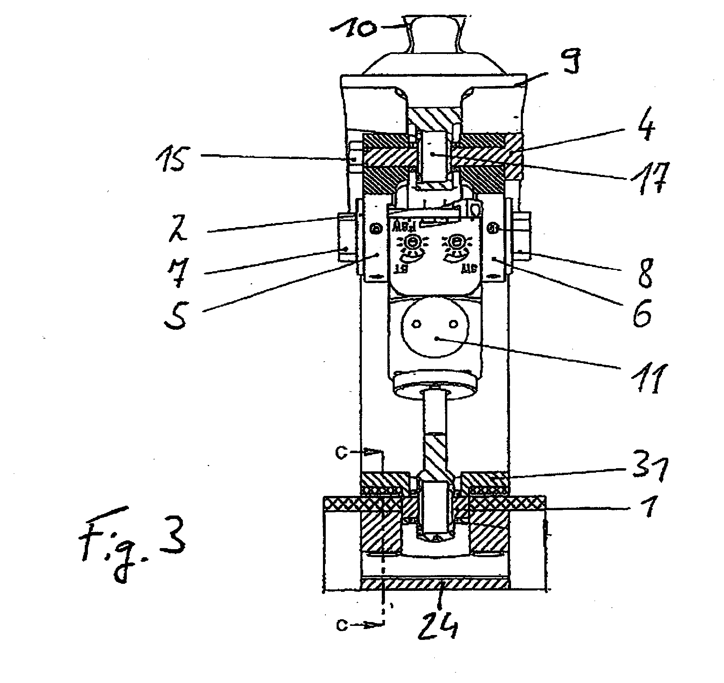

In der Figur 2 ist eine Seitenansicht des Prothesenfußes 100 gemäß Figur 1 mit einem Detailschnitt entlang der Linie C-C in Figur 3 dargestellt. In der Seitenansicht gemäß Figur 2 ist zunächst die im Wesentlichen gerade, im Vorderfußbereich 231 leicht gekrümmte Ausgestaltung der Basisfeder 23 zu erkennen. Das Torsionselement 28 als Blattfeder liegt in dem Auflagerbereich 260 über einem Pufferelement 30 auf der Oberseite der Basisfeder 23 auf. Das Pufferelement 30 erstreckt sich über die gesamte Breite des Torsionselementes 28. Zwischen dem Torsionselement 28 und der Basisfeder 23 ist ein Freiraum 33 jenseits des Pufferelementes 30 vorgesehen. Das Pufferelement 30 besteht vorzugsweise aus einem Kunststoff, insbesondere aus Polyurethan.

Der Figur 2 ist ebenfalls zu entnehmen, dass das untere Ende 250 der Frontalabstützung 25, 26, 27 an dem Torsionselement 28 befestigt ist. Die Frontalabstützung 25, 26, 27 ist im dargestellten Ausführungsbeispiel aus drei gebogenen Blattfederelementen 25, 26, 27 ausgebildet. Zwischen den einzelnen Blattfederelementen 25, 26, 27 ist ein Freiraum vorhanden, der beispielsweise durch Distanzstücke 22 am oberen Ende 140 der Frontalabstützung 25, 26, 27 sichergestellt wird. Distanzstücke können ebenfalls am unteren Ende 250 der Frontalabstützung zwischen den Blattfederelementen 25, 26, 27 angeordnet sein.

Das obere Ende der Frontalabstützung 25, 26, 27 ist über Schrauben 14 an dem Proximaladapter 9 befestigt. Zwischen den Schrauben 14 und dem frontalen Blattfederelement 27 ist ein Druckblech 19 vorgesehen, um die Kräfte der Schrauben 14 gleichmäßig auf das frontale Blattfederelement 27 zu übertragen.

Der Hydraulikdämpfer 11 ist an dem rückwärtigen Bereich des Proximaladapters 9 befestigt. Das untere Ende des Hydraulikdämpfers 11 ist über einen Fersenarm 31 im Fersenbereich 232 der Basisfeder 23 festgelegt. Der Fersenarm 31 ist über Zylinderschrauben 12 an der Basisfeder 23 befestigt. Die Zylinderschrauben 12 gehen durch die Basisfeder 23, eine Distanzscheibe 21 zur Gewährleistung des Freiraums 33 zwischen der Basisfeder 23 und dem Torsionselement 28, und dem Torsionselement 28 selbst hindurch und werden in den Fersenarm 31 eingeschraubt. Über die Zylinderschrauben 12 wird gleichzeitig das Dämpferelement 32 an der Basisfeder 23 festgelegt. Über Zylinderschrauben 13 und ein Druckblech 18 wird die Fersenfeder 24 an dem Dämpferelement 32 befestigt. Durch die Schraubverbindung kann eine auswechselbare Ausgestaltung der Fersenfeder 24 realisiert werden, wodurch eine weitere Anpassung an den jeweiligen Prothesenfußnutzer erfolgen kann. Statt eines Dämpferelementes 32 kann auch eine Adapterplatte vorgesehen sein, über die die Fersenfeder 24 an der Basisfeder 23 festgelegt werden kann. Die Adapterplatte kann als Abstützung für der Hydraulikdämpfer 11 dienen. Durch eine gerundete Anlagefläche für die Fersenfeder 24 kann eine zunehmende Erhöhung der Widerstandskraft erreicht werden, da sich die wirksame Länge der Fersenfeder 24 bei zunehmender Belastung verringert.

In der Figur 3 ist ein Schnitt entlang der Linie B-B in Figur 2 dargestellt. Es ist eine teilgeschnittene Rückansicht des Prothesenfußes mit dem Justierkern 10 und dem Proximaladapter 9 zu erkennen. Zwei Haltearme 5, 6 sind über ein Gelenklager 17 und einen Gelenkbolzen 4 mit einer Mutter 15 gelenkig an dem Proximaladapter 9 gelagert. An den beiden Haltearmen 5, 6 ist über Schrauben 7, 8 der Hydraulikdämpfer 11 befestigt. Der Fersenarm 31 nimmt einen Adapterbolzen 1 auf, der zur gelenkigen Lagerung des Hydraulikdämpfers 11 ausgebildet ist. Die Fersenfeder 24 bildet den unteren Abschluss des Prothesenfußes 100.

Wir der Prothesenfuß 100 in der dargestellten Ausführungsform eingesetzt, tritt zunächst die Fersenfeder 24 in Kontakt mit dem Boden. Eine elastische Verformung tritt ein. Kräfte werden über die Schrauben 13 und das Dämpferelement 32 auf die Basisfeder 23 übertragen. Aufgrund der einseitigen Einspannung durch die Schrauben 12 des Torsionselementes 28 hebt sich das vordere Ende in dem Abstützbereich 260 von der Basisfeder 23 ab. Der Hydraulikdämpfer 11 wird zusammengedrückt und die Frontalabstützung 25, 26, 27 wird mit dem oberen Ende 140 nach hinten gebogen. Durch das Anheben des vorderen Endes des Torsionselementes 28 und die Tordierbarkeit um die Prothesenfußlängsachse 230 ist es möglich, dass eine Torsions- und Kippbewegung beim Auftreten durchgeführt wird, so dass die Basisfeder 23 und die Fersenfeder 24 sich an einen unebenen Boden anpassen können.

Nach dem Fersenstoß biegen sich das Torsionselement 28 sowie die Blattfederelemente 25, 26, 27 der Frontalabstützung zurück in ihre Ausgangsposition, der Hydraulikdämpfer 11 wird in Richtung auf seine Ausgangsposition zurückbewegt. Das vordere, untere Ende der Frontalabstützung 25, 26, 27 liegt dann unter Zwischenschaltung des Torsionselements 28 auf der Basisfeder 23 über das Pufferelement 30 auf. Bei einer weiteren Abrollbewegung wird eine zunehmende Last auf den Vorderfußbereich 231 aufgebracht. Die Blattfederelemente 25, 26, 27 biegen sich weiter durch, so dass die Krümmung verstärkt wird. Das Torsionselement 28, die Blattfederelemente 25, 26, 27 und die Basisfeder 23 wirken dann zusammen, wobei die Basisfeder 23 und das Torsionselement 28 als Blattfeder parallel geschaltet sind. Aufgrund der lediglich einseitigen Einspannung kann das Torsionselement 28 bei der Durchbiegung der Basisfeder 23 auf deren Oberfläche verschoben werden. Die Frontalabstützung kann aus einem Blattfederpaket aus Blattfederelementen 25, 26, 27 oder einem einzelnen Blattfederelement bestehen. Eine Torsion um eine Vertikalachse 250 ist aufgrund der konstruktiven Ausgestaltung sowohl der Abstützung des Hydraulikdämpfers 11 als auch der Frontalabstützung 25, 26, 27 möglich.

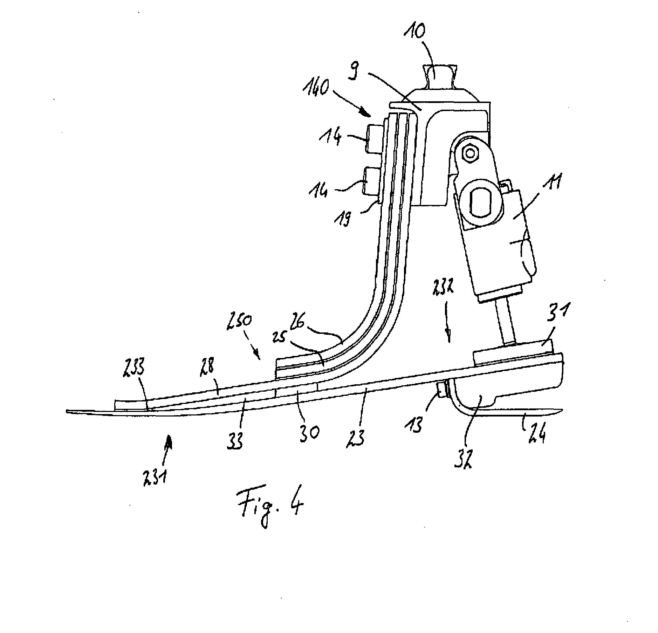

In der Figur 4 ist eine Variante der Erfindung in Seitenansicht dargestellt. Der grundsätzliche Aufbau entspricht dem gemäß Figur 2, statt eines im Fersenbereich 332 der Basisfeder 23 befestigten Torsionselementes 28 ist dieses in der Variante im Vorderfußbereich 231 an der Basisfeder 23 festgelegt. Das Torsionselement 28 ist unmittelbar nach dem Zehenbereich der Basisfeder 23 an dieser festgelegt, im dargestellten Ausführungsbeispiel über eine Klebestelle 233 oder eine Verschweißung oder andere Art und Weise der Festlegung des Torsionselementes 28 an der Basisfeder 23. Zwischen der Befestigungsstelle 233 und dem Pufferelement 30 ist ein Freiraum 33 ausgebildet, das Pufferelement 30 ermöglicht eine Torsionsbewegung des Torsionselementes 28 relativ zu der Basisfeder 23. Das Torsionselement 28 verläuft von der Befestigungsstelle 233 nach einer leichten Aufweitung des Zwischenraumes 33 im Wesentlichen parallel zu der Basisfeder 23 bis nach dem Pufferelement 30 und von dort in einem Bogen, der in eine Gerade mündet, zu dem Proximaladapter 9, wo es über die Schrauben 14 an dem oberen Ende 140 der Frontalabstützung 25, 26 befestigt ist. Die Frontalabstützung 25, 26 ist als eine Anordnung im Wesentlichen parallel verlaufender Blattfedern ausgebildet, der Verlauf der Frontalabstützung 25, 26 entspricht im Wesentlichen dem Verlauf des Torsionselementes 28 ab dem Pufferelement 30 in Richtung auf den Proximaladapter 9. Zwischen der Frontalabstützung 25, 26 und dem Torsionselement 28 sind jeweils Freiräume ausgebildet, um eine Verbiegung und eine Relativbewegung der Blattfedern 26, 26, 28 relativ zueinander während des Gehens oder Stehens zu ermöglichen.

Das Pufferelement 30 ist entweder an der Basisfeder 23 oder an dem Torsionselement 28 befestigt, so dass bei einem harten Fersenauftritt und einer Aufbiegung des Torsionselementes 28 dieses sich relativ von der Basisfeder 23 wegbewegen kann.

Das vordere Ende 250 der Frontalabstützung 25, 26 endet ungefähr auf Höhe des Pufferelementes 30 zwischen dem Torsionselement 28 und der Basisfeder 23.

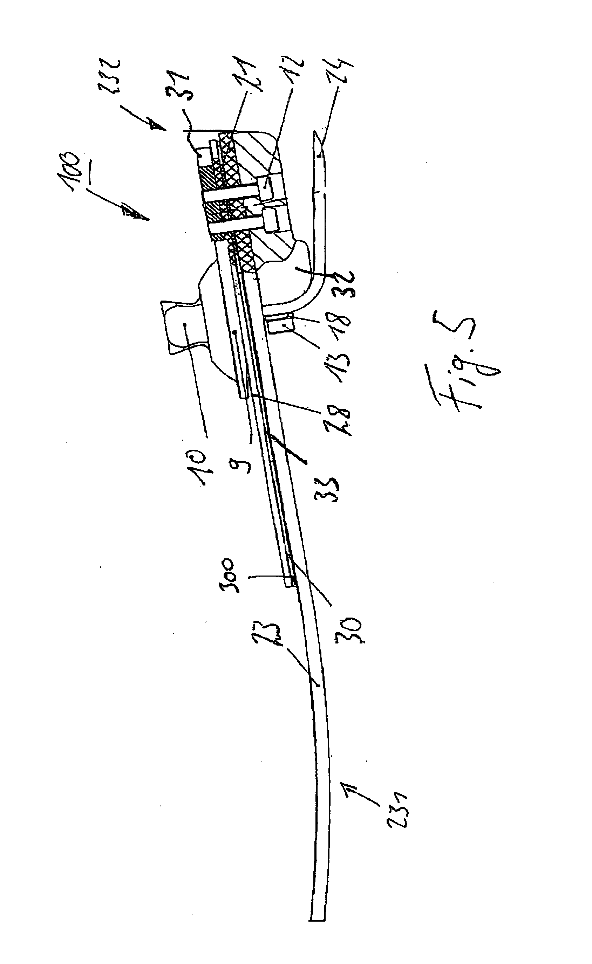

In der Figur 5 ist eine Variante ohne Frontalabstützung dargestellt. Der Prothesenfuß 100 weist analog der Figuren 1 und 2 einen Vorderfußbereich 231 und einen Fersenbereich 232 auf. Auch hier ist die Basisfeder 23 aus einem Faserverbundwerkstoff hergestellt und die Ausführungen zu der Basisfeder 23 und zu dem oberhalb der Basisfeder 23 angeordneten Torsionselement 28 geltend entsprechend. Ein Freiraum 33 ist zwischen dem Torsionselement 28 und der Oberseite der Basisfeder 23 angeordnet. Am vorderen Ende des Torsionselementes 28 kann ein Polsterelement 300 angeordnet sein, um störende Geräusche bei einer Relativbewegung zwischen dem Torsionselement 28 und der Basisfeder 23 zu verhindern.

Das Torsionselement 28 ist an seinem hinteren Ende an dem hinteren Ende der Basisfeder 23 im Fersenbereich 232 über die Schrauben 12 einseitig festgelegt. Die Befestigung erfolgt ausschließlich im Fersenbereich 232 der Basisfeder 23. Die Anschlusseinrichtung aus Proximaladapter 9 und Justierkern 10 ist auf dem Torsionselement 30 in Gehrichtung vor den Befestigungselementen 12 angeordnet und gegebenenfalls mit einem Keil unterlegt, um eine Neigungseinstellung und dadurch eine Anpassung der Ausrichtung oberer Prothesenkomponenten zu erleichtern. Durch die direkte Anbindung der Anschlusseinrichtung an das Torsionselement 28 und dessen einseitige Lagerung kann neben einer Parallelschaltung des Torsionselementes 28 mit der Basisfeder 23 auch eine Verlagerbarkeit bzw. Verkippbarkeit der nicht dargestellten oberen Anschlussmittel um die Fußlängsachse erfolgen. Ebenfalls kann das Torsionselement 28 als zusätzliche Feder dienen und eine Parallelschaltung zwischen der Basisfeder 23 und dem Torsionselement 28 bewirken.

## Patentansprüche

1. Prothesenfuß mit einer Basisfeder (23) mit einem Vorderfußbereich (231) und einem Fersenbereich (232), einer oberhalb der Basisfeder (23) angeordneten Anschlusseinrichtung (9, 10) zur Befestigung des Prothesenfußes an einer Prothese und einer Frontalabstützung (25, 26, 27), an deren oberen Ende (140) die Anschlusseinrichtung (9, 10) angeordnet und an ihrem unteren Ende (250) an einem um die Prothesenfußlängsachse (230) tordierbaren Torsionselement (28) festgelegt ist, wobei das Torsionselement (28) als eine einseitig im Fersenbereich (232) oder im Vorderfußbereich (231) an der Basisfeder (23) festgelegte Blattfedereinrichtung ausgebildet ist, **dadurch gekennzeichnet, dass** an der Anschlusseinrichtung (9, 10) oder an dem oberen Ende (140) der Frontalabstützung (25, 26, 27) ein Dämpferelement (11) oder ein nachgiebiges Element angeordnet ist, das sich im Fersenbereich (232) der Basisfeder (23) an der Basisfeder (23) oder dem Torsionselement (28) abstützt.

2. Prothesenfuß nach Anspruch 1, **dadurch gekennzeichnet, dass** zwischen der Basisfeder (23) und dem Torsionselement (28) ein Freiraum (33) ausgebildet ist.

3. Prothesenfuß nach Anspruch 1 oder 2, **dadurch gekennzeichnet, dass** zwischen dem Torsionselement (28) und der Basisfeder (23) ein Pufferelement (30) angeordnet ist.

4. Prothesenfuß nach einem der voranstehenden Ansprüche, **dadurch gekennzeichnet, dass** das Torsionselement (28) um eine medial-lateral-Achse (240) tordierbar ist.

5. Prothesenfuß nach einem der voranstehenden Ansprüche, **dadurch gekennzeichnet, dass** an der Basisfeder (23) eine Fersenfeder (24) angeordnet ist.

6. Prothesenfuß nach Anspruch 5, **dadurch gekennzeichnet, dass** zwischen der Basisfeder (23) und der Fersenfeder (24) ein Dämpferelement (32) angeordnet ist.

7. Prothesenfuß nach einem der voranstehenden Ansprüche, **dadurch gekennzeichnet, dass** die Frontalabstützung (25, 26, 27) als Feder oder Federpaket ausgebildet ist.

8. Prothesenfuß nach einem der voranstehenden Ansprüche, **dadurch gekennzeichnet, dass** die Frontalabstützung (25, 26, 27) um die Vertikalachse (250) tordierbar ausgebildet ist.

9. Prothesenfuß nach einem der voranstehenden Ansprüche, **dadurch gekennzeichnet, dass** das Dämpferelement (11) als Fluiddämpfer ausgebildet ist.

10. Prothesenfuß nach einem der voranstehenden Ansprüche, **dadurch gekennzeichnet, dass** das Dämpferelement (11) zumindest ein mehrachsig bewegliches Gelenk (34) aufweist.

## Claims

1. A prosthetic foot with a base spring (23) with a forefoot region (231) and a heel region (232), a connection apparatus (9, 10), arranged above the base spring (23), for affixing the prosthetic foot to a prosthesis and a frontal support (25, 26, 27), at the upper end (140) of which the connection apparatus (9, 10) is arranged and the lower end (250) of which is secured to a torsion element (28) which can be twisted about the prostheticfoot longitudinal axis (230), whereas the torsion element (28) is configured as a leaf spring apparatus, secured at one end to the base spring (23), either in the heel region (232) or in the forefoot region (231) **characterized in that** a damping element (11) or a resilient element, which is supported on the base spring (23) or on the torsion element (28) in the heel region (232) of the base spring (23), is arranged on the connection apparatus (9, 10) or on the upper end (140) of the frontal support (25, 26, 27).

2. The prosthetic foot as claimed in claim 1, **characterized in that** a clear space (33) is formed between the base spring (23) and the torsion element (28).

3. The prosthetic foot as claimed in claims 1 or 2, **characterized in that** a buffer element (30) is arranged between the torsion element (28) and the base spring (23).

4. The prosthetic foot as claimed in one of the preceding claims, **characterized in that** the torsion element (28) can be twisted about a medial/lateral axis (240).

5. The prosthetic foot as claimed in one of the preceding claims, **characterized in that** a heel spring (24) is arranged on the base spring (23).

6. The prosthetic foot as claimed in claim 5, **characterized in that** a damping element (32) is arranged between the base spring (23) and the heel spring (24).

7. The prosthetic foot as claimed in one of the preceding claims, **characterized in that** the frontal support (25, 26, 27) is configured as a spring or spring package.

8. The prosthetic foot as claimed in one of the preceding claims, **characterized in that** the frontal support (25, 26, 27) is configured in such a way that it can be twisted about the vertical axis (250).

9. The prosthetic foot as claimed in one of the preceding claims, **characterized in that** the damping element (11) is configured as a fluid damper.

10. The prosthetic foot as claimed in one of the preceding claims, **characterized in that** the damping element (11) has at least one multi-axially movable joint (34).

## Revendications

1. Pied prothétique comprenant un ressort de base (23) avec une zone d'avant-pied (231) et une zone de talon (232), un dispositif de raccordement (9, 10) agencé au-dessus du ressort de base (24) pour la fixation du pied prothétique sur une prothèse, et un soutien frontal (25,26, 27) à l'extrémité supérieure duquel (140) est agencé le dispositif de raccordement (9, 10) et à l'extrémité inférieure duquel (250) est immobilisé un élément de torsion (28) susceptible d'être tordu autour de l'axe longitudinal du pied prothétique (230), dans lequel l'élément de torsion (28) est réalisé sous la forme d'un système de ressort à lame immobilisé sur le ressort de base (23) d'un seul côté dans la zone de talon (232) ou dans la zone d'avant-pied (231),
**caractérisé en ce qu'**un élément amortisseur (11) ou un élément flexible est agencé sur le dispositif de raccordement (9, 10) ou sur l'extrémité supérieure (140) du soutien frontal (25, 26, 27), élément qui s'appuie dans la zone de talon (232) du ressort de base (23) sur le ressort de base (23) ou sur l'élément de torsion (28).

2. Pied prothétique selon la revendication 1, **caractérisé en ce qu'**un espace libre (33) est ménagé entre le ressort de base (23) et l'élément de torsion (28).

3. Pied prothétique selon la revendication 1 ou 2, **caractérisé en ce qu'**un élément tampon (30) est agencé entre l'élément de torsion (28) et le ressort de base (23).

4. Pied prothétique selon l'une des revendications précédentes, **caractérisé en ce que** l'élément de torsion (28) est susceptible d'être tordu autour d'un axe médial-latéral (240).

5. Pied prothétique selon l'une des revendications précédentes, **caractérisé en ce qu'**un ressort de talon (24) est agencé sur le ressort de base (23).

6. Pied prothétique selon la revendication 5, **caractérisé en ce qu'**un élément amortisseur (32) est agencé entre le ressort de base (23) et le ressort de talon (24).

7. Pied prothétique selon l'une des revendications précédentes, **caractérisé en ce que** le soutien frontal (25, 26, 27) est réalisé comme un ressort ou comme un empilement de ressorts.

8. Pied prothétique selon l'une des revendications précédentes, **caractérisé en ce que** le soutien frontal (25, 26, 27) est réalisé de manière à pouvoir être tordu autour de l'axe vertical (250).

9. Pied prothétique selon l'une des revendications précédentes, **caractérisé en ce que** l'élément amortisseur (11) est réalisé comme un amortisseur à fluide.

10. Pied prothétique selon l'une des revendications précédentes, **caractérisé en ce que** l'élément amortisseur (11) comprend au moins une articulation (34) mobile suivant plusieurs axes.
